# EUROPEAN PATENT APPLICATION

(11) **EP 4 431 611 A1**
(43) Date of publication of application: **18.09.2024**
(21) Application number: 23162645.8
(22) Date of filing: 17.03.2023
(51) Int. Cl.: C12P 13/02, C12N 9/18

(54) **AN ENZYMATIC PROCESS FOR PRODUCING HYDROXYCINNAMIC ACID AMIDES (HCAA)**

(71) Applicant: Analyticon Discovery GmbH, 14473 Potsdam (DE)
(72) Inventor: Siems, Karsten, 14552 Michendorf (DE); Baumert, Benjamin, 17489 Greifswald (DE); Bornscheuer, Uwe, 17489 Greifswald (DE)
(74) Representative: Fabry, Bernd

(57) **Abstract**

Suggested is an enzymatic process for preparing hydroxycinnamic acid amides comprising or consisting of the following steps:
(i) providing at least one hydroxycinnamic acid ester (component a);
(ii) providing at least one amine (component b);
(iii) providing at least one source of *Pyrobaculum calidifontis* VA1 esterase (component c);
(iv) providing an aqueous buffer (component d)
(v) blending components (a) to (d) to form a reaction mixture;
(vi) adjusting the pH to a value above 7;
(vii) incubating the reaction mixture at a temperature ranging from about 25 to about 90°C for preferably at least 5 minutes; and
(viii) collecting the hydroxycinnamic acid amides thus obtained from the reaction mixture.

## Description

### AREA OF INVENTION

The present invention refers to the area of active agents from nature and a method for enzymatic synthesis of hydroxycinnamic acid amides under aqueous conditions.

### BACKGROUND OF THE INVENTION

Hydroxycinnamic acid amides (HCAAs) are a naturally occurring, diverse class of secondary metabolites with presumptive roles in plant growth, development and senescence. HCAAs have many positive effects on the human body, e.g. due to their antioxidant, antidiabetic, cytotoxic and anti-cancer, neuroprotective, anti-inflammation properties and effects on inflammatory related diseases as well as skin health and anti-melanogenesis activity (William Leonard , Pangzhen Zhang , Danyang Ying & Zhongxiang Fang (2020): Tyramine-derived hydroxycinnamic acid amides in plant foods: sources, synthesis, health effects and potential applications in food industry, Critical Reviews in Food Science and Nutrition. https://doi.org/10.1080/10408398.2020.1845603). HCCAs are grouped into two major classes according to their amine moiety:
- HCCA with an aromatic amine, e.g., tyramine, dopamine, tryptamine, or serotonin
- HCCAs with an aliphatic amine, e.g., putrescine or spermidine

Examples of HCCAs are provided in the following figure:

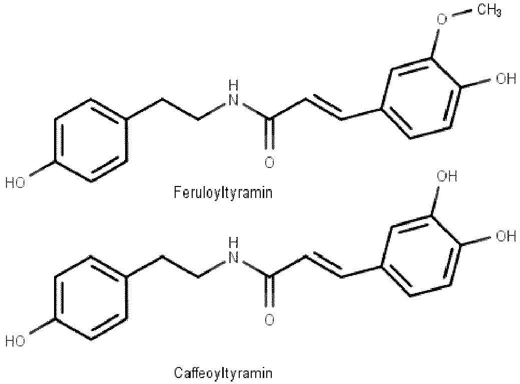

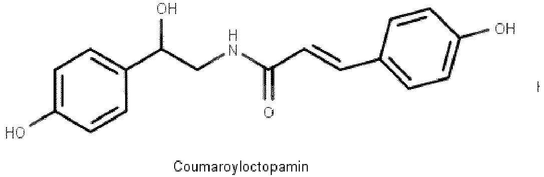

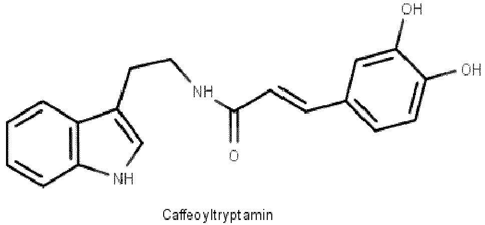

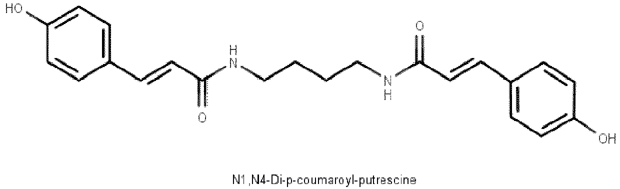

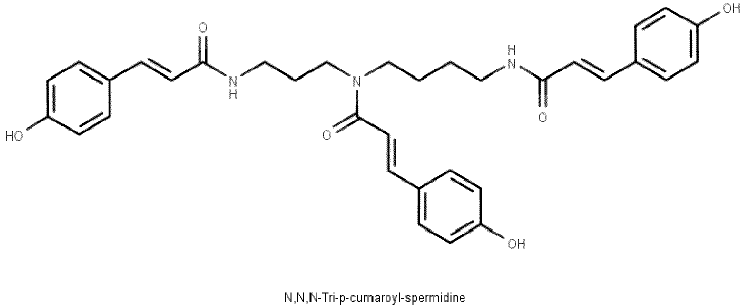

HCCAs are occurring in many plants, but usually in small amounts only. Therefore, direct extraction from plants is not an economically feasible production process. Chemical synthesis is cheap and easy by condensation of amines with activated cinnamic acids (e.g., as acyl chlorides or using catalysts like dicyclohexyl carbodiimide DCCI), but there is a demand from industry and final customers for *natural* ingredients. According to REGULATION (EC) No 1334/2008 (Article 3 2.c) ‴natural flavoring substance shall mean a flavoring substance obtained by appropriate physical, enzymatic or microbiological processes from material of vegetable, animal or microbiological origin". Compounds produced by chemical synthesis are not allowed to be labelled as *natural,* whereas enzymatically produced compounds may be labelled as *natural.* Additionally, food industry is highly interested in sustainable production methods. Some enzymatic reactions are running in organic solvents. According to the principles of *Green Chemistry* reactions in aqueous media are preferred.

### RELEVANT PRIOR ART

### Enzymatic synthesis of amides

There are a number of enzymes that catalyze the synthesis of amides. These enzymes can be divided into two groups. The first group consists of ATP-dependent enzymes that use ATP to activate carboxylic acids'. Due to the use of expensive ATP these enzymes are not very interesting for industrial processes. The second group consists of hydrolases for example lipases, esterases and acylases². The majority of these enzymes also require activated carboxylic acids in the form of esters. Another advantage is that these enzymes do not require cofactors and many of them are very robust, which makes them excellent for industrial applications³. Lipases and esterases retain their activity in water-free organic solvents, which allows them to catalyze amide bond formation. The company BASF uses amide bond formation catalyzed by lipases as the basis for kinetic separation of racemic amines⁴. However the use of organic solvents has several drawbacks, including high costs, negative environmental impact and incompatibility with food and cosmetic applications due to toxicity³. Moreover, these reactions must be performed in the absence of water in pure organic solvents or an organic mixture of the reactants to achieve product formation in the (trans-)esterification and to avoid undesired hydrolysis of the product formed. However, there are hydrolases that maintain aminolysis activity in water, so called promiscuous hydrolases/acyltransferases³. The best studied example is a hydrolase from *Mycobacterium smegmatis* (MsAcT). This enzyme has been used for the synthesis of simple amides with acyl donors like vinyl acetate or ethyl acetate^{5,6}, but more recently also for more structurally complex molecules like vanillamides in water (yield 5-50% with 2x access of amine)⁷. Müller et al. discovered the Family VIII Carboxylesterase EstCE1 as another promiscuous hydrolase/acyltransferase that catalyzes amide bond formation. This enzyme shows that this type of reaction is not limited to MsAcT. In addition they could show that EstCE1's catalytic potential goes beyond structurally simple compounds by the synthesis of the antidepressant moclobemide⁸.

### Pyrobaculum calidifontis VA1 esterase (PestE)

Discovered by Hotta et al.⁹ **PestE** is an extremely stable Carboxylesterase from a hyperthermophilic archaeon with a pH-optimum of pH 7 for hydrolase activity and a temperature optimum of 70 °C. Its promiscuous hydrolase/acyltransferase activity was discovered by Müller et al.¹⁰ Its acyltransferase activity for the formation of ester bonds was improved by enzyme engineering^{11,12}.

### Enzymatic synthesis of N-trans-feruloyl tyramine and N-trans-caffeoyl tyramine with lipases in organic solvents

*N*-trans-caffeoyltyramine and *N*-trans-feruloyltyramine and other coumaroyl tyramine derivatives were previously synthesized using an immobilized *Candida antartica* lipase B from Novozym (Novozym 435)¹³. They use as substrates methyl esters of the corresponding carboxylic acids and tyramine as nucleophile with a ratio of 1:2 and MTBE as solvent at 50 °C for 24h reaching conversions of 77.5% and 73.1% respectively. Conversions were determined by measuring the decrease of substrate by HPLC¹³.

Alrub et al. show the synthesis of *N*-trans-Feruloyl tyramine carried out at 40 °C for 24 h using various lipases in dry acetonitrile and ferulic acid and tyramine-hydrochloride as substrates in a ratio of 1:1 reaching approx. 69.9% conversion measured by HPLC¹⁴.In both examples care must be taken that the system is non-aqueous to achieve ester product formation and to avoid undesired hydrolysis.

### Enzymatic synthesis of amides in aqueous solution

Land et al. used the promiscuous hydrolase/acyltransferase MsAcT in a cascade reaction with a transaminase and discovered that the optimal pH for the aminolysis reaction was at pH 11 and they concluded this is due to the protonation state of the nucleophile benzylamine with a p*K*ₐ of 9.34¹⁵. They did only use methyl acetate or methyl methoxyacetate as acyl donor but no hydroxyl cinnamic acids.

### OBJECT OF THE INVENTION

Therefore, it has been the object of the present invention providing a method for the enzymatic synthesis of natural hydroxycinnamic acid amides (HCAAs) in high conversions and high yields under aqueous conditions, so that the products are allowed to be labelled "natural".

### BRIEF DESCRIPTION OF THE INVENTION

The present invention refers to an enzymatic process for preparing hydroxycinnamic acid amides comprising or consisting of the following steps:
(i) providing at least one hydroxycinnamic acid ester (component a);
(ii) providing at least one amine (component b);
(iii) providing at least one source of *Pyrobaculum calidifontis* VA1 esterase ("PestE", component c);
(iv) providing an aqueous buffer (component d)
(v) blending components (a) to (d) to form a reaction mixture;
(vi) adjusting the pH to a value above 7;
(vii) incubating the reaction mixture at a temperature ranging from about 25 to about 90 °C for preferably at least 5 minutes; and
(viii) collecting the hydroxycinnamic acid amides thus obtained from the reaction mixture.

Surprisingly, it has been observed that carboxylesterases of the PestE type are efficient enzymatic catalysts for the formation of hydroxycinnamic acid amides (HCAA) from hydroxycinnamic acid esters and various types of amines. Amidation takes place under mild conditions in aqueous medium and provides in short times both high conversion and high yields. Since the HCAA are obtained from an enzymatic process the products are allowed to be labelled "natural".

### Hydroxycinnamic acid esters

Hydroxycinnamic acid esters (component a) represent esters of hydroxycinnamic acids with C₁-C₆ aliphatic, aromatic, saturated or unsaturated alcohols. Preferred esters are obtained from methanol, ethanol, propanol, butanol and particularly allyl alcohol. They can be obtained by standard esterification processes which are known to persons skilled in the art.

Hydroxycinnamic acids (hydroxycinnamates) are a class of aromatic acids or phenylpropanoids having a C₆-C₃ skeleton. These compounds are hydroxy derivatives of cinnamic acid. 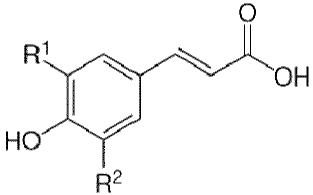 in which R1 and R2 independently from each other can represent hydrogen, hydroxyl, or C₁-C₄ alkoxy.
- Caffeic acid - e.g., from burdock, hawthorn, artichoke, pear, basil, thyme, oregano, apple;
- Cichoric acid;
- Cinnamic acid - e.g., from aloe;
- Chlorogenic acid - e.g., from echinacea, strawberries, pineapple, coffee, sunflower, blueberries;
- Diferulic acids;
- Coumaric acid;
- Ferulic acid - e.g., from oats, rice, artichoke, orange, pineapple, apple, peanut; and
- Sinapic acid

Preferably, component (a) is selected from the group consisting of esters of ferulic acid, caffeic acid, coumaric acid and mixtures thereof. More preferably, component (a) represents a methyl, ethyl or vinyl ester of ferulic acid, caffeic acid, coumaric acid and mixtures thereof.

### Amines

The nature of the amines (component b) is not critical and fully depends on the type of HCAA which is desired. To react with the hydroxycinnamic acid esters suitable amines require at least one nitrogen with an acidic hydrogen. Therefore, suitable amines encompass primary and secondary amines. Also suitable are polyamines, such as diamines or triamines, from which HCAA are obtained having two or more hydroxycinnamic acid groups.

Preferably, component (b) represents an aliphatic, cycloaliphatic or aromatic (poly) amine having 3 to 20 carbon atoms. More preferably, component (b) is selected from the group consisting of tyramine, dopamine, tryptamine, octopamine, serotonin, putrescine, spermidine and mixtures thereof.

Overall preferred is an embodiment wherein methyl, ethyl or vinyl esters of ferulic acid, caffeic acid, coumaric acid and mixtures thereof are reacted with tyramine, dopamine, tryptamine, octopamine, serotonin, putrescine, spermidine and mixtures thereof.

### Pyrobaculum calidifontis VA1 esterase

PestE (component c) is a synonym for the highly thermostable esterase from the hyperthermophilic archaeon *Pyrobaculum calidifontis* VA1, which shows high enantioselectivity (E > 100) in the kinetic resolution of racemic chiral carboxylic acids, but little selectivity towards acetates of tertiary alcohols (E = 2-4). The enzyme is a member of the hormonesensitive lipase group (group H) of the esterase/lipase superfamily on the basis of the amino acid sequence identity. The PestE structure shows a canonical α/β-hydrolase fold as core domain with a cap structure at the C-terminal end of the β-sheet.

Component (c) can be selected from the group consisting of the following PestE variants or mixtures thereof:
- N288A
- L209F
- G86A
- G86A_I208A_L209F
- I208F
- G86A_I208A
- G86A_N288A
- L209F_N288A
- I208A
- I208A_L209F
- I208A_N288A
- G86A_L209F
- I208A_L209F_N288A

### Buffers

While standard phosphate buffers are not working well, the preferred alternatives for component (d) encompass CHES buffer (*N*-cyclohexyl-2-aminoethanesulfonic acid) or CAPS buffer (*N*-cyclohexyl-3-aminopropanesulfonic acid) 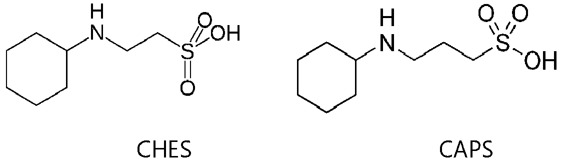 maintaining a pH of about 9.5 to 11.5. In the alternative it also possible using an amine with a pKa-Wert of less than seven, given that the pH value is high enough to allow deprotonation of the amine.

### Reaction conditions

In a first step, components (a) to (d) are mixed and adjusted to a pH value of from preferably about 8 to about 12.5, more preferably from about 9 to about 11.5 for example by adding NaOH. If necessary, additional water is also added to form the reaction mixture. Subsequently the mixture is incubated at a preferred temperature of from about 30 to about 90 °C, and preferably from about 50 to about 70 °C over a period of about 5 minutes to about 48 hours, preferably about 30 minutes to about 24 hours and more preferably from about 60 minutes to about 15 hours.

In a particularly preferred embodiment components (a) and (b) are reacted in the presence of at least one PestE variant (for example obtained by recombinant expression in E. coli, used as crude cell lysate or in purified form) selected from the group consisting of I208A_L209F, I208A_N288A, G86A_L209F, or I208A_L209F_N288A, preferably at a pH of from 10 to 11 and a temperature of from about 50 to about 80 °C. The PestE variants identified above represent the overall preferred enzymes for the reaction. They are providing conversions and yields up to 100 % of theory. The enzymes can be obtained by methods known to a person skilled in the art, they are also available in the market.

For the sake of good order, it should be emphasized that also other modified esterases from the hyperthermophilic archaeon *Pyrobaculum calidifontis* VA1 are covered by the present invention. Further modified variants are considered representing full equivalents of the variants disclosed above, in case they provide HCAE in yields and/or conversions of up to 50 % of the theory and particularly of about 70 to about 100 % of the theory. This is typically the case for variants showing a sequence homology - compared to the preferred PestE variants disclosed above - of at least 80 %, preferably about 70 to 95 % and more preferably about 80 to 90 %.

### Collection of the HCAA

For collecting the hydroxycinnamic acid amides the reaction mixture can be subjected to extraction. A particular preferred extraction agent is ethyl acetate. It is advantageous extracting the reaction mixture, two or three times, combining the organic phases and evaporating the solvent to obtain the desired HCAA.

### EXAMPLES

### Example 1

### Development and lab-scale N-trans-feruloyl tyramine synthesis with PestE

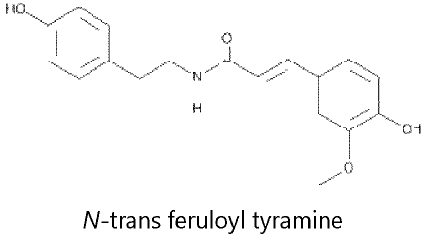

### A) Preparation of lyophilized E. coli lysates with overexpressed promiscuous acyl transferases

Lyophilized *E. coli* lysates with promiscuous acyl transferases were prepared according to Godehard et al.¹⁶

### B) Screening of promiscuous acyl transferases for N-trans-feruloyl tyramine synthesis

5 mg lyophilized *E. coli* lysates with overexpressed promiscuous acyl transferases were resuspended in 200 mM potassium phosphate buffer pH 8. 2 M vinyl ferulate was dissolved in acetonitrile. Tyramine was dissolved in 200 mM potassium phosphate buffer, pH 8. Due to the addition of tyramine the pH shifts to pH 10. Final reaction concentrations: 44.5 mM tyramine, 178 mM potassium phosphate buffer, 100 mM vinyl ferulate, 0.3 mg/ml lyophilized lysate, 5 % (v/v) acetonitrile. Lysate from *E. coli* cultivations with empty vector served as control. Incubation for 16 h at 25 °C and 1000 rpm. Tested enzymes: 1EVQ, 3FAK, 4XVC, 3K6K, 5GMX, 4IVI, Est24, Est8, MsAcT, EstA, EstCE1_MAA, EstCE1, WP061, WP039, WP007, WP084, GCD93, PestE, PestE_I208A, PestE_L209F. Samples were analyzed using HPLC.

EstCE1_MAA, GCD93, PestE_I208A and PestE_L209F showed significantly higher N-trans-feruloyl tyramine formation than control and PestE_I208A showed by far the highest N-trans-feruloyl tyramine formation.

### C) Gene expression and protein purification of Pyrobaculum calidifontis VA1 Esterase (PestE) variants

Expression and purification of PestE variants were conducted as described previously¹⁷ with the following modifications: E. *coli* were cultivated in ZYM-5052-medium for protein expression and the purified enzymes were not mixed with 0.1% Triton-X-100 for storage at 4 °C.

Briefly: *E*. *coli* BL21(DE3) cells were transformed with the pET-21a expression vector containing PestE-genes. Pre-cultures (4 mL LB containing 100 µg·mL⁻¹ ampicillin) were inoculated with single colonies and incubated overnight (37 °C, 180 rpm). ZYM-5052 medium according to Studier et al.¹⁸ with 100 µg·mL⁻¹ ampicillin was inoculated with 0.1% (v/v) of the pre-culture and incubated (37 _{°}C, 180 rpm) for 2 h and subsequently incubated for 20 h at 20 °C at 180 rpm. Cells were harvested by centrifugation at 4000x g and 4 °C for 15 min, and the cell pellets were resuspended with 4 mL equilibration buffer (50 mM potassium phosphate, 300 mM sodium chloride, 10 mM imidazole, pH 8.0). Cells were disrupted by sonication on ice (two cycles of 5 min sonication (30% intensity, 50% pulsed cycle)) using a SONOPULS HD 2070 (BANDELIN Electronic GmbH & Co. KG, Berlin, Germany), and the lysates were clarified by centrifugation at 10,000x g and 4 °C for 30 min. For purification, the crude lysates were applied to 1.5 mL Roti^{®} Garose-His/Ni Beads (Carl Roth, Karlsruhe, Germany). The resins were washed with 15 mL washing buffer (50 mM potassium phosphate, 300 mM sodium chloride, 20 mM imidazole, pH 8.0) before target proteins were eluted with elution buffer (50 mM potassium phosphate, 300 mM sodium chloride, 250 mM imidazole, pH 8.0). Elution fractions were treated at 80 °C for 20 min (500 rpm), centrifuged (17,000x g, 4 °C for 5 min), and the supernatant transferred to the storage buffer (50 mM potassium phosphate, 300 mM sodium chloride, pH 8.0) using PD-10 desalting columns (GE Healthcare, Chalfont St Giles, UK). Protein concentrations were determined at 280 nm using a NanoDrop^{™} 1000 spectrophotometer (ThermoFisher, Darmstadt, Germany), while the purity of the proteins was investigated by SDS-PAGE.

### D) Test of pH-optimum

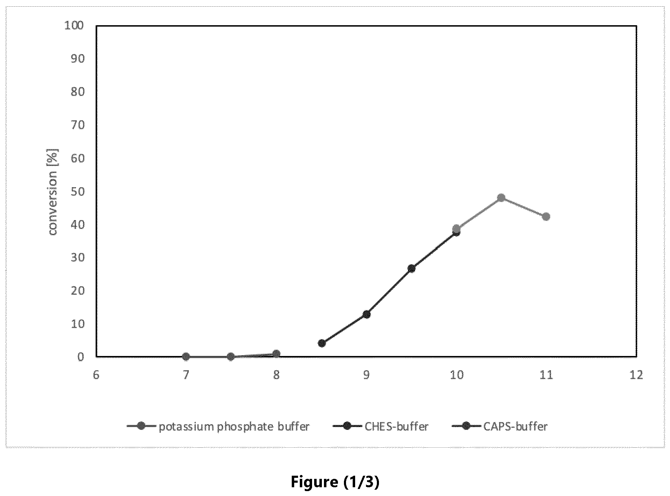

5 mM tyramine was dissolved in 200 mM potassium phosphate buffer, CHES buffer or CAPS buffer and the pH was adjusted to pH 7, 7.5 and 8 for phosphate buffer, pH 8.5, 9, 9.5 and 10 for CHES buffer and pH 10, 10.5 and 11 for CAPS buffer. Stock solution: 200 mM vinyl ferulate in acetonitrile. Final concentration: 4.45 mM tyramine, 178 mM buffer, 10 mM vinyl ferulate, 5% acetonitrile, 0.21 mg/ml PestE_I208A. Samples were incubated at 25 °C measured on HPLC after depletion of vinyl ferulate. The results are shown in **Table 1** and **Figure 1****.**

**Table 1**

| Influence of pH on the synthesis of N-trans-feruloyl tyramine with PestE_I208A (5 mM tyramine and 10 mM vinyl ferulate). | | |
|---|---|---|
| **Buffer** | **pH** | **Conversion [%]** |
| potassium phosphate | 7.0 | 0.00 |
| potassium phosphate | 7.5 | 0.00 |
| potassium phosphate | 8.0 | 0.92 |
| CHES buffer | 8.5 | 4.13 |
| CHES buffer | 9.0 | 12.94 |
| CHES buffer | 9.5 | 26.66 |
| CHES buffer | 10.0 | 37.65 |
| CAPS buffer | 10.0 | 38.59 |
| CAPS buffer | 10.5 | 47.97 |
| CAPS buffer | 11.0 | 42.27 |

### E) Test of PestE variants

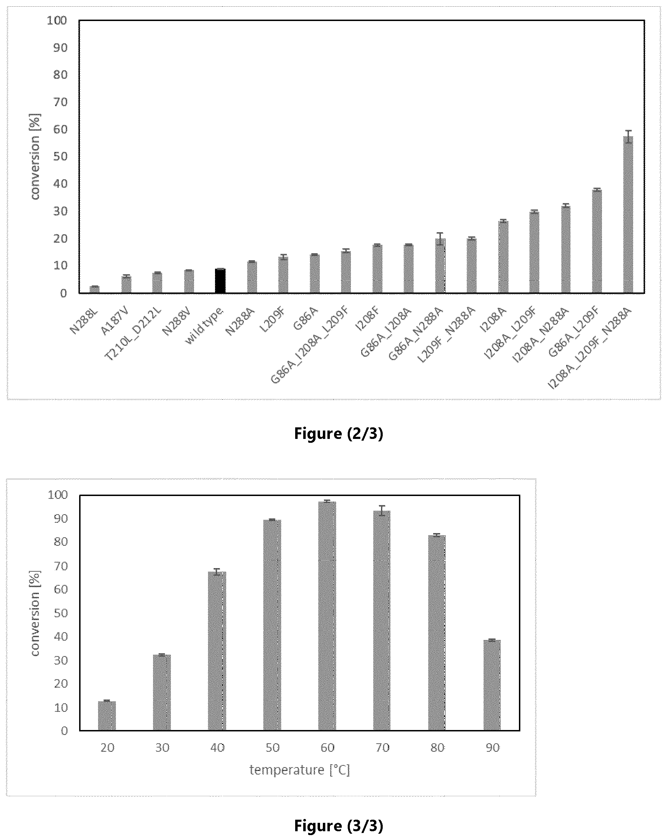

Tyramine was dissolved in 200 mM CHES buffer and pH was adjusted with HCl or NaOH to pH 10. Stock solution: 100 mM vinyl ferulate in acetonitrile. Final concentration: 5 mM tyramine, 178 mM CHES-buffer, 5 mM methyl ferulate, 5% acetonitrile, 0.21 mg/ml PestE variant. Samples were incubated at 25 °C at 1000 rpm and triplicates were measured by HPLC after full conversion of vinyl ferulate. The results are shown in **Table 2** and **Figure 2****.**

**Table 2**

| *N*-trans-feruloyl tyramine synthesis with different PestE variants (5 mM tyramine and 5 mM vinyl ferulate) | | |
|---|---|---|
| **Variant** | **Conversion [%]** | **Standard deviation [%]** |
| N288L | 2.57 | ±0.08 |
| A187V | 6.03 | ±0.58 |
| T210L_D212L | 7.24 | ±0.28 |
| N288V | 8.24 | ±0.16 |
| wild type | 8.74 | ±0.20 |
| N288A | 11.35 | ±0.22 |
| L209F | 13.13 | ±0.87 |
| G86A | 13.95 | ±0.22 |
| G86A_I208A_L209F | 15.39 | ±0.57 |
| I208F | 17.60 | ±0.40 |
| G86A_I208A | 17.63 | ±0.14 |
| G86A_N288A | 19.85 | ±2.12 |
| L209F_N288A | 19.93 | ±0.44 |
| I208A | 26.41 | ±0.44 |
| I208A_L209F | 29.78 | ±0.57 |
| I208A_N288A | 31.98 | ±0.71 |
| G86A_L209F | 37.79 | ±0.61 |
| I208A_L209F_N288A | 57.34 | ±2.19 |

### F) Test of temperature optimum

Tyramine was dissolved in 200 mM CHES buffer and pH was adjusted with HCl or NaOH to pH 10. Stock solution: 200 mM methyl ferulate in acetonitrile. Final concentration: 5 mM tyramine, 178 mM CHES-buffer, 10 mM methyl ferulate, 5% acetonitrile, 0.21 mg/ml PestE_I208A_L209F_N288A. Samples were incubated at 25 °C, 30 °C, 40 °C, 50 °C, 60 °C, 70 °C, 80 °C and 90 °C and triplicates were measured by HPLC after 2 h incubation. At 70 °C CHES buffer at pH 10 seems to be the best condition. The results are shown in **Table** 3 and **Figure 3****.**

**Table 3**

| *N*-trans-feruloyl tyramine synthesis with methyl ferulate as acyl-donor and conversion after 2 h (5 mM tyramine and 10 mM methyl ferulate) | | |
|---|---|---|
| **Temperature [°C]** | **Conversion [%]** | **Standard deviation [%]** |
| 20 | 12.76 | |
| 30 | 32.30 | |
| 40 | 67.56 | |
| 50 | 89.48 | ±2.99 |
| 60 | 97.39 | ±1.52 |
| 70 | 93.39 | ±4.23 |
| 80 | 83.06 | ±2.37 |
| 90 | 38.60 | ±1.48 |

### G) Test of Acyl donors

Tyramine was dissolved in 200 mM CHES buffer and pH adjusted with HCl or NaOH to pH 10. Acyl donor stock solution: 100 mM vinyl ferulate, methyl ferulate or ethyl ferulate in acetonitrile. Final concentration: 5 mM tyramine, 178 mM CHES-buffer, 5 mM methyl ferulate, 5% acetonitrile, 0.21 mg/ml PestE_I208A_L209F_N288A. Samples were incubated at 70 °C at 1000 rpm and triplicates were measured on HPLC after full conversion of ferulic acid ester. The results are shown in **Table 4.**

**Table 4**

| *N*-trans-feruloyl tyramine synthesis with different acyl-donors (5 mM tyramine and 5 mM acyl-donor) | | |
|---|---|---|
| **Acyl-donor** | **Conversion [%]** | **Standard deviation [%]** |
| Ethyl ferulate | 58.16 | ±3.24 |
| Methyl ferulate | 63.21 | ±1.82 |
| Vinyl ferulate | 62.10 | ±0.55 |

### H) Test of the ratio of acyl donor to acyl acceptor

Tyramine was dissolved in 200 mM CHES buffer and pH was adjusted with HCl or NaOH to pH 10. Acyl donor stock solution: 100 mM or 200 mM methyl ferulate in acetonitrile. Final concentration: 5 mM or 10 mM tyramine, 178 mM CHES-buffer, 5 mM methyl ferulate, 5% acetonitrile, 0.21 mg/ml PestE_I208A_L209F_N288A. Samples were incubated at 70 °C at 1000 rpm and triplicates were measured on HPLC until full conversion of ferulic acid ester. The results are shown in **Table 5.**

**Table 5**

| *N*-trans-feruloyl tyramine synthesis with different ratios of acyl-donor and acyl-acceptor | | |
|---|---|---|
| **Ratio of acyl donor to acyl acceptor** | **Conversion [%]** | **Standard deviation [%]** |
| Methyl ferulate 10 mM und 5 mM Tyramine | 95.91 | ±3.08 |
| 5 mM Methyl ferulate 10 mM Tyramine | 91.07 | ±6.06 |

### J) Scale-up and isolation of the final product

21.9 mg tyramine, 70.9 mg methyl ferulate, 1.32 g CHES were dissolved in water and the pH was adjusted to pH 10 with NaOH. 6.68 mg PestE_I208A_L209F_N288A were added and the volume was adjusted to 32 mL in a falcon. The sample was incubated until methyl ferulate was depleted. *N*-trans-feruloyl tyramine was extracted three times with ethyl acetate. The organic phase was washed with brine, dried over anhydrous Mg₂SO₄, filtered and the solvent was evaporated under reduced pressure. The crude product (40 mg of crude product means 80 % yield) was purified by flash chromatography. ¹H-NMR analysis showed the desired product.

### Brief description of the drawings

- Figure 1:: Influence of pH on the synthesis of *N*-trans-feruloyl tyramine with PestE_I208A (5 mM tyramine and 10 mM vinyl ferulate).
- Figure 2:: *N*-trans-feruloyl tyramine synthesis with different PestE variants (5 mM tyramine and 5 mM vinyl ferulate)
- Figure 3:: *N*-trans-feruloyl tyramine synthesis with methyl ferulate as acyl-donor and conversion after 2 h (5 mM tyramine and 10 mM methyl ferulate).

### CITED REFERENCES

(1) Petchey, M. R.; Grogan, G. Enzyme-Catalysed Synthesis of Secondary and Tertiary Amides. Adv. Synth. Catal. 2019, 361 (17), 3895-3914. https://doi.org/10.1002/adsc.201900694.
(2) Wu, S.; Snajdrova, R.; Moore, J. C.; Baldenius, K.; Bornscheuer, U. T. Biocatalysis: Enzymatic Synthesis for Industrial Applications. Angew. Chem. Int. Ed. 2021, 60 (1), 88-119. https://doi.org/10.1002/anie.202006648.
(3) Müller, H.; Terholsen, H.; Godehard, S. P.; Badenhorst, C. P. S.; Bornscheuer, U. T. Recent Insights and Future Perspectives on Promiscuous Hydrolases/Acyltransferases. ACS Catal. 2021, 14906-14915. https://doi.org/10.1021/acscatal.1c04543.
(4) Ditrich, K. Optically Active Amines by Enzyme-Catalyzed Kinetic Resolution. Synthesis 2008, 2283-2287. https://doi.org/10.1055/s-2008-1078451.
(5) Contente, M. L.; Farris, S.; Tamborini, L.; Molinari, F.; Paradisi, F. Flow-Based Enzymatic Synthesis of Melatonin and Other High Value Tryptamine Derivatives: A Five-Minute Intensified Process. Green Chem. 2019, 21 (12), 3263-3266. https://doi.org/10.1039/C9GC01374A.
(6) Contente, M. L.; Pinto, A.; Molinari, F.; Paradisi, F. Biocatalytic N-Acylation of Amines in Water Using an Acyltransferase from Mycobacterium Smegmatis. Adv. Synth. Catal. 2018, 360 (24), 4814-4819. https://doi.org/10.1002/adsc.201801061.
(7) Pinna, C.; Martino, P. A.; Meroni, G.; Sora, V. M.; Tamborini, L.; Dallavalle, S.; Contente, M. L.; Pinto, A. Biocatalyzed Synthesis of Vanillamides and Evaluation of Their Antimicrobial Activity. J. Agric. Food Chem. 2022, 70 (1), 223-228. https://doi.org/10.1021/acs.jafc.1c06213.
(8) Müller, H.; Godehard, S. P.; Palm, G. J.; Berndt, L.; Badenhorst, C. P. S.; Becker, A.; Lammers, M.; Bornscheuer, U. T. Discovery and Design of Family VIII Carboxylesterases as Highly Efficient Acyltransferases. Angew. Chem. Int. Ed. 2021, 60 (4), 2013-2017. https://doi.org/10.1002/anie.202014169.
(9) Hotta, Y.; Ezaki, S.; Atomi, H.; Imanaka, T. Extremely Stable and Versatile Carboxylesterase from a Hyperthermophilic Archaeon. Appl. Environ. Microbiol. 2002, 68 (8), 3925-3931. https://doi.org/10.1128/AEM.68.8.3925-3931.2002.
(10) Müller, H.; Becker, A.; Palm, G. J.; Berndt, L.; Badenhorst, C. P. S.; Godehard, S. P.; Reisky, L.; Lammers, M.; Bornscheuer, U. T. Sequence-Based Prediction of Promiscuous Acyltransferase Activity in Hydrolases. Angew. Chem. 2020, 132 (28), 11704-11709. https://doi.org/10.1002/ange.202003635.
(11) Staudt, A.; Terholsen, H.; Kaur, J.; Müller, H.; Godehard, S. P.; Itabaiana, I.; Leal, I. C. R.; Bornscheuer, U. T. Rational Design for Enhanced Acyltransferase Activity in Water Catalyzed by the Pyrobaculum Calidifontis VA1 Esterase. Microorganisms 2021, 9 (8), 1790. https://doi.org/10.3390/microorganisms9081790.
(12) Terholsen, H.; Kaur, J.; Kaloudis, N.; Staudt, A.; Müller, H.; Pavlidis, I. V.; Bornscheuer, U. T. Recovery of Hydroxytyrosol from Olive Mill Wastewater Using the Promiscuous Hydrolase/Acyltransferase PestE. ChemBioChem 2022. https://doi.org/10.1002/cbic.202200254.
(13) Zeng, F.; Zhang, H.; Xu, M.; Huang, K.; Zhang, T.; Duan, J. Immobilized Lipase Catalytic Synthesis of Phenolamides and Their Potential against α-Glucosidase. J. Biotechnol. 2021, 334, 51-57. https://doi.org/10.1016/jjbiotec.2021.04.002.
(14) Alrub, M. A.; Basri, M.; Malek, E. A.; Alang Ahmad, S. A.; Salleh, A. B.; Abdul Rahman, M. B. Lipase Catalysed Synthesis of N-Trans-Feruloyltyramine and a Quantitative HPLC-UV Method for Analysis. Biocatal. Biotransformation 2012, 30 (4), 385-390. https://doi.org/10.3109/10242422.2012.701623.
(15) Land, H.; Hendil-Forssell, P.; Martinelle, M.; Berglund, P. One-Pot Biocatalytic Amine Transaminase/Acyl Transferase Cascade for Aqueous Formation of Amides from Aldehydes or Ketones. Catal. Sci. Technol. 2016, 6 (9), 2897-2900. https://doi.org/10.1039/C6CY00435K.
(16) Godehard, S. P.; Badenhorst, C. P. S.; Müller, H.; Bornscheuer, U. T. Protein Engineering for Enhanced Acyltransferase Activity, Substrate Scope, and Selectivity of the Mycobacterium Smegmatis Acyltransferase MsAcT. ACS Catal. 2020, 10 (14), 7552-7562. https://doi.org/10.1021/acscatal.0c01767.
(17) Staudt, A.; Terholsen, H.; Kaur, J.; Müller, H.; Godehard, S. P.; Itabaiana, I.; Leal, I. C. R.; Bornscheuer, U. T. Rational Design for Enhanced Acyltransferase Activity in Water Catalyzed by the Pyrobaculum Calidifontis VA1 Esterase. Microorganisms 2021, 9 (8), 1790. https://doi.org/10.3390/microorganisms9081790.
(18) Studier, F. W. Protein Production by Auto-Induction in High-Density Shaking Cultures. Protein Expression and Purification 2005, 41 (1), 207-234. https://doi.org/10.1016/j.pep.2005.01.016.

## Claims

**1.** An enzymatic process for preparing hydroxycinnamic acid amides comprising or consisting of the following steps:
(i) providing at least one hydroxycinnamic acid ester (component a);
(ii) providing at least one amine (component b);
(iii) providing at least one source of *Pyrobaculum calidifontis* VA1 esterase (component c);
(iv) providing an aqueous buffer (component d)
(v) blending components (a) to (d) to form a reaction mixture;
(vi) adjusting the pH to a value above 7;
(vii) incubating the reaction mixture at a temperature ranging from about 25 to about 90 °C for preferably at least 5 minutes; and
(viii) collecting the hydroxycinnamic acid amides thus obtained from the reaction mixture.

**2.** The process according to Claim 1, wherein component (a) is selected from the group consisting of esters of hydroxycinnamic acids with C₁-C₆ aliphatic, aromatic, saturated or unsaturated alcohols.

**3.** The process according to Claim 1, wherein component (a) is selected from the group consisting of esters of caffeic acid, cichoric acid, cinnamic acid, chlorogenic acid, diferulic acids, coumaric acid, ferulic acid, sinapic acid and mixtures thereof.

**4.** The process according to Claim 2 and/or 3, wherein component (a) represents a methyl, ethyl or vinyl ester of ferulic acid, caffeic acid, coumaric acid and mixtures thereof.

**5.** The process according to Claim 1, wherein component (b) represents a primary amine, a secondary amine, a polyamine or a mixture thereof.

**6.** The process according to Claim 5, wherein component (b) represents an aliphatic, cycloaliphatic or aromatic amine having 3 to 20 carbon atoms.

**7.** The process according to Claim 5 and/or 6, wherein component (b) is selected from the group consisting of tyramine, dopamine, tryptamine, octopamine, serotonin, putrescine, spermidine and mixtures thereof.

**8.** The process according to any of the preceding claims 1 to 7, wherein methyl, ethyl or vinyl esters of ferulic acid, caffeic acid, coumaric acid and mixtures thereof are reacted with tyramine, dopamine, tryptamine, octopamine, serotonin, putrescine, spermidine and mixtures thereof.

**9.** The process according to any of the preceding claims 1 to 8, wherein component (c) is selected from the group consisting of the following PestE variants or mixtures thereof:
• N288A
• L209F
• G86A
• G86A_I208A_L209F
• I208F
• G86A_I208A
• G86A_N288A
• L209F_N288A
• I208A
• I208A_L209F
• I208A_N288A
• G86A_L209F
• I208A_L209F_N288A

**10.** The process according to any of the preceding claims 1 to 9, wherein component (d) represents a CHES buffer or a CAPS buffer.

**12.** The process according to any of the preceding claims 1 to 10, wherein the pH is adjusted to a value of from about 8 to about 12.5.

**12.** The process according to any of the preceding claims 1 to 11, wherein the incubation is conducted at a temperature of from about 30 to about 90 °C.

**13.** The process according to any of the preceding claims 1 to 12, wherein the incubation is conducted over a period of from about 3 to about 48 hours.

**14.** The process according to any of the preceding claims 1 to 12, wherein components (a) and (b) are reacted in the presence of at least one PestE variant selected from the group consisting of I208A_L209F, I208A_N288A, G86A_L209F, or I208A_L209F_N288A, at a pH of from 10 to 11 and a temperature of from about 50 to about 80 °C.

**15.** The process according to any of the preceding claims 1 to 14, wherein the hydroxycinnamic acid amides are collected from the reaction mixture by extraction.
